(19) Europäisches Patentamt
European Patent Office
Office européen des brevets

(11) Publication number: 0 006 738 B2

(12) NEW EUROPEAN PATENT SPECIFICATION

(45) Date of publication of the new patent specification: 16.09.87

(51) Int. Cl.⁴: A 61 K 7/38

(21) Application number: 79301202.2

(22) Date of filing: 21.06.79

# (54) Antiperspirants.

(30) Priority: 23.06.78 GB 2775678

(43) Date of publication of application: 09.01.80 Bulletin 80/1

(45) Publication of the grant of the patent: 29.09.82 Bulletin 82/39

(45) Mention of the opposition decision: 16.09.87 Bulletin 87/38

(84) Designated Contracting States: DE FR GB

(56) References cited:
DE - A - 2 700 711
FR - A - 2 202 527
FR - A - 2 259 587
FR - A - 2 278 319
FR - A - 2 303 527
US - A - 2 814 584
US - A - 3 211 620
US - A - 3 904 741

(73) Proprietor: UNILEVER PLC, Unilever House Blackfriars P.O. Box 68, London EC4P 4BQ (GB)
(84) Designated Contracting States: GB

(73) Proprietor: UNILEVER NV, Burgemeester s'Jacobplein 1 P.O. Box 760, NL-3000 DK Rotterdam (NL)
(84) Designated Contracting States: DE FR

(72) Inventor: Pike, Barry Graham, 8 Tanglewood, Finchampstead Wokingham, Berkshire (GB)

(74) Representative: Doucy, Robert Henry et al, Unilever PLC Patents Division P.O. Box 68 Unilever House, London EC4P 4BQ (GB)

EP 0 006 738 B2

## Description

This invention relates to antiperspirant compositions.

For inhibiting perspiration, the application to the skin of many different antiperspirant active compounds has been described in the literature. However, those compounds most widely used in commercial products at the present time are basic aluminium halides, especially aluminium chlorhydrate, which has an Al/Cl molar ratio of about 2. These active compounds are applied to the skin from a variety of applicator types including aerosol sprays, pump sprays, squeeze packs, roll-ons and stick applicators. Thus aluminium chlorhydrate, for example, is employed as the active ingredient of various liquid, cream, stick or dry powder antiperspirant compositions. However, in spite of the popularity of aluminium chlorhydrate the presently available products are capable of producing only limited reduction in perspiration.

In our DE-A 2 700 711, referred to hereinafter as the first application, there is described an improved antiperspirant active compound which comprises a basic aluminium chloride, bromide, iodide and nitrate having an aluminium to chloride, bromide, iodide or nitrate molar ratio of from 6.5 to 1.3:1, and which compound forms in water a solution containing polymeric species of a size greater than 100 Angstroms within which species there is contained at least 2% by weight of the total aluminium. Since in aqueous solutions of the basic aluminium compounds the halide or nitrate is in ionic form the polymeric species present are hydroxyaluminium species. The antiperspirant active compound may be employed in the form of an aqueous solution or the solution may be spray dried to give a hydrated compound of the empirical formula:

$$Al_2(OH)_{6-a}X_a \cdot nH_2O$$

where X is Cl, Br, I or $NO_3$, a is from about 0.3 to 1.5 and n is from about 0.3 to 8.

As described in said first application, these special forms of basic aluminium compounds which in aqueous solution contain polymeric species having a size greater than 100 Angstroms within which at least 2% by weight of the total aluminium is contained, may be prepared by heating aqueous solutions of basic aluminium compounds at elevated temperature. The production of the desired species depends on the appropriate choice of the reaction conditions which are interrelated. It is preferred to use temperatures of from 80°C to 140°C. The period of heating may be shorter as higher temperatures are used, ranging for example from 0.5 hour to 30 days. Of importance is the concentration of the basic aluminium compound starting material. The rate of production of the higher polymeric species of the basic aluminium compound decreases as the concentration of the solution is increased and at the above temperatures the concentration should be no more than about 35% by weight.

In our DE-A 2 818 321, referred to herein as the second application, we have described an improvement in aqueous solutions of the antiperspirant agents of the first application. This improvement consisted in the inclusion in the solution of a neutral amino acid, by which was meant an amino acid containing an equal number of unneutralised amino and acidic groups. By the inclusion of the amino acid reduction of solution viscosity is effected and gelling inhibited, particularly of aqueous alcoholic solutions.

We have now found that these advantages can also be obtained through the inclusion of urea in the solution.

Before continuing with a description of the present invention, we would explain that it is not novel per se to include urea in a composition comprising an aluminium antiperspirant compound. Three patent specifications mentioned in the search report which disclose the inclusion of urea in a composition comprising an aluminium antiperspirant composition are referred to below.

In US-A 2 814 584 there is described an antiperspirant composition comprising in combination an aqueous solution of a zirconium or hafnium salt of a strong monobasic mineral acid, a basic aluminium compound and urea. The basic aluminium compound and the urea act as buffering agents to bring the pH of the solution of the zirconium or hafnium salt to a value which renders it safe for antiperspirant usage. The urea is present in an amount of 5 to 50% by weight of the composition on an anhydrous basis and sufficient to prevent gelling of the composition.

In US-A 3 211 620 there is described a composition comprising neomycin, a nitrogen-containing organic compound which normally tends to inactivate neomycin upon ageing in an aqueous medium at elevated temperatures and a soluble aluminium compound in an amount to inhibit such inactivation. The composition can also include from 0.1 to 5% by weight of urea to reduce corrosion or discolouration upon the ironing of clothing fabric due to the application of an aluminium astringent compound.

In FR-A 2 303 527 there is described a method of making an antiperspirant composition in powder form which comprises spray drying an aqueous solution of a mixture of urea, aluminium chloride and aluminium chlorhydrate. Inclusion of the urea reduces skin irritation caused by the aluminium compounds.

According to the present invention therefore there is provided an antiperspirant composition comprising an aqueous solution of a basic aluminium chloride, bromide, iodide or nitrate having an aluminium to chloride, bromide, iodide or nitrate molar ratio of from 6.5 to 1.3:1 which solution contains polymeric hydroxyaluminium species derived from the basic halogenide or nitrate of a size greater than 100 Angstroms within which there is contained from 2 to 80% by weight of the

total aluminium, and which solution also contains urea as a gelation inhibitor.

The amount of the urea included in the aqueous solution, which may be an aqueous alcoholic solution, of the basic aluminium compound is preferably such that the aluminium to urea molar ratio is from 20:1 to 1:10. The amount of urea necessary to substantially inhibit gelation will depend on the concentration of the basic aluminium compound in the solution and on the amount of any alcohol present, the higher concentrations of the basic aluminium compound and/or alcohol requiring the use of greater amounts of urea.

The basic aluminium compound preferably has an aluminium to chloride, bromide, iodide or nitrate molar ratio of from 4 to 1.3:1, more particularly to 2.5 to 1.6:1.

The weight of aluminium in the species having an effective diameter above 100 Angstroms is preferably from 5 to 60% of the total aluminium.

The aqueous solution of the active antiperspirant agent comprising the higher polymeric species as defined, may, if desired, be evaporated to concentrate the solution or it may be dried to give a solid hydrated material. As with ordinary aluminium chlorhydrate, for example, drying conditions which lead to both the loss of water of condensation and hydrochloric acid should be avoided as these may lead to irreversible degradation of the basic aluminium compound. Any suitable method of drying may be used, spray drying being a particularly useful method. The spray drying method described in US Patent No. 3 887 692 may be employed. The solid material may be ground or milled as required. Drying should be effected in such manner as to give a product having a water content consistent with the following empirical formula:

$$Al_2(OH)_{6-a}X_a \cdot nH_2O \cdot (urea)_m$$

where X is Cl, Br, I or $NO_3$, a is from about 0.31 to 1.5, n is from about 0.3 to 8, preferably 0.3 to 4, and m indicates the amount of urea and is preferably about 0.1 to 20.

The compositions of the invention may be made by dissolving the urea in the solution of the basic aluminium compound containing the polymeric species greater than 100 Angstroms prepared as described in the first application referred to above. Alternatively the urea may be included in a solution made up from the powder obtained by drying the solution prepared according to the said first application referred to above. A further method is by dissolving the solid, urea-containing antiperspirant compounds described in the preceding paragraph in water or a water-alcohol mixture.

The compositions of the invention comprising the basic aluminium compound and urea may contain up to 75% by weight of a $C_1$–$C_3$ aliphatic alcohol. These aqueous alcoholic compositions preferably contain ethanol, propanol, isopropanol or a mixture thereof as the alcohol. The basic aluminium compound is desirably present in the aqueous or aqueous alcoholic solution in a concentration within the range 1 to 25% by weight.

The antiperspirant composition of the invention may be applied to the skin in various known ways. Thus the aqueous solution may be applied from a pump spray applicator or from a roll-ball applicator. Conventional ingredients may be included in such compositions, for example, perfume (usually from 0.1 to 2% by weight) or thickener (usually 0.1 to 5% by weight).

The following Examples illustrate the invention. Percentages are by weight.

Examples 1 and 2

A spray dried antiperspirant powder was made according to Example 2 of the first application referred to above. According to this Example aluminium chlorhydrate having an Al/Cl molar ratio of 2.04 and a water content of 18.5% was dissolved in deionised water to give a 10% w/w solution. This solution was heated in 1 litre screw-cap glass bottles to 97–100°C over 10 hours and then held at this temperature for a further 38 hours. The resulting solution was cooled to room temperature and found to contain 23.9% of the total aluminium as polymers exceeding 100 Angstroms in effective diameter. The solution was then spray dried in a co-current spray drier using inlet and outlet temperatures of 250°C and 95°C, respectively. The powder had an Al/Cl molar ratio of 2.14 and a water content of 14.3%. Aqueous alcoholic solutions of this material and urea were made up. They were prepared by mixing ethanol with the powder to form a slurry to which was added the urea followed by the water. The amounts of the ingredients used are indicated below.

| | Example 1 % | Example 2 % |
|---|---|---|
| Antiperspirantpowder | 10 | 15 |
| Ethanol | 40 | 30 |
| Urea | 15 | 10 |
| Distilled water | 35 | 45 |

In each case the urea prevented gelation of the solution.

Example 3

An aqueous solution of the antiperspirant material used in Examples 1 and 2 was made by dissolving 15 parts of urea of water after which 20 parts of the antiperspirant powder were stirred in. The urea prevented gelation of the solution.

Example 4

120 g of urea were dissolved in 2 kg of the treated solution made according to Example 9 of the first application referred to above. According to this Example 140 kg of an aluminium chlorhydrate solution (Reheis "Chlorhydrol") having an Al/Cl

ratio of 2.09 was diluted with 560 litres of deionised water at 45 °C and stirred and heated to 120 °C in a stainless steel reactor over 3.75 hours.

Stirring and heating at this temperature was maintained for a further 5.5 hours before cooling rapidly to 70 °C and more slowly to ambient temperature. The resulting solution was found to contain 30.6% of the total aluminium as polymers greater than 100 Angstroms in effective diameter. The treated solution containing the urea was dried in a cocurrent spray drier using inlet and outlet temperatures of 250°C and 95°C, respectively. The resulting powder had the following empirical formula:

$$Al_2(OH)_{5.1}Cl_{0.9}(H_2O)_{0.4}(urea)_{1.9}$$

A further sample of the treated solution to which no urea had been added was similarly spray dried to give a powder having the empirical formula:

$$Al_2(OH)_{5.1}Cl_{0.9}(H_2O)_{1.5}$$

Solutions of these powders in aqueous alcohol having the same aluminium concentration were prepared from the following ingredients:

| | % | % |
|---|---|---|
| Powder containing urea | 20 | - |
| Powder not containing urea | - | 15 |
| Water | 60 | 65 |
| Ethanol | 20 | 20 |

The solutions were prepared by mixing the ethanol with the respective powder to form a slurry to which was added the water. Stirring was maintained throughout the process.

The solution obtained using the powder containing urea showed no sign of gel formation after 1 week whereas the other solution had gelled.

## Claims

1. An antiperspirant composition comprising an aqueous solution of basic aluminium chloride, bromide, iodide or nitrate having an aluminium to chloride, bromide, iodide or nitrate molar ratio of from 6.5 to 1.3:1 which solution contains polymeric hydroxyaluminium species derived from the basic aluminium halogenide or nitrate of a size greater than 100 Angstroms within which there is contained from 2 to 80% by weight of the total aluminium, and which solution also contains urea as a gelation inhibitor.

2. An antiperspirant composition as claimed in Claim 1 wherein the amount of urea is such that the aluminium to urea molar ratio is from 20:1 to 1:10.

3. An antiperspirant composition as claimed in Claim 1 or Claim 2 wherein the solution is an aqueous alcoholic solution comprising a $C_1$–$C_3$ aliphatic alcohol in an amount up to 75% by weight.

4. An antiperspirant composition as claimed in any of the preceding claims comprising from 1 to 25% by weight of the basic aluminium compound.

5. A solid antiperspirant composition produced by drying the aqueous composition claimed in Claim 1 or Claim 2 and having the empirical formula:

$$Al_2(OH)_{6-a}X_a \cdot nH_2O \cdot (urea)m$$

where X is Cl, Br, I or $NO_3$, a is from 0.31 to 1.5, n is from 0.3 to 8, and m indicates the amount of urea.

## Patentansprüche

1. Antischweissmittel bestehend aus einer wässrigen Lösung eines basischen Aluminiumchlorides, -bromides, -jodides oder -nitrates mit einem Aluminium zu Chlorid, bzw. Bromid bzw. Jodid bzw. Nitrat Molarverhältnis von 6,5–1,3:1, wobei die Lösung polymeres Hydroxyaluminium enthält, abgeleitet von dem basischen Aluminiumhalogenid oder -nitrat von einer Grösse grösser als 100 Å worin 2–80 Gewichtsprozent des Gesamtaluminiums enthalten sind, und wobei die Lösung auch Harnstoff als Gelierungsinhibitor enthält.

2. Antischweissmittel gemäss Anspruch 1, in welchem die Harnstoffmenge derart ist, dass dass Aluminium zu Harnstoff Molarverhältnis 20:1–1:10 beträgt.

3. Antischweissmittel gemäss Anspruch 1 oder 2, bei welchem die Lösung eine wässrige alkoholische Lösung mit einem $C_1$–$C_3$ aliphatischen Alkohol in einer Menge bis zu 75 Gewichtsprozent ist.

4. Antischweissmittel gemäss einem der vorgehenden Ansprüche enthaltend 1–25 Gewichtsprozent der basischen Aluminiumverbindung.

5. Festes Antischweissmittel hergestellt durch Trocknen des wässrigen Mittels gemäss Anspruch 1 oder 2 und mit der empirischen Formel:

$$Al_2(OH)_{6-a}X_a \cdot nH_2O \cdot (Harnstoff)m$$

in welcher X Cl, Br, J oder $NO_3$ bedeutet, a 0,31–1,5 beträgt, n 0,3–8 beträgt und m die Menge an Harnstoff anzeigt.

## Revendications

1. Composition antiperspirante comprenant une solution aqueuse d'un chlorure, bromure, iodure ou nitrate d'aluminium basique ayant un rapport molaire de l'aluminium au chlorure, bromure, iodure, ou nitrate allant de 6,5 à 1,3:1, solution qui contient une espèce d'hydroxyaluminium polymérique dérivée de l'halogénure ou nitrate d'aluminium basique d'une taille supérieure à 100 Angström dans laquelle se trouve de 2 à 80% en poids de l'aluminium total, solution qui contient

également de l'urée comme inhibiteur de gélification.

2. Composition antiperspirante selon la revendication 1 où la quantité d'urée est telle que le rapport molaire de l'aluminium à l'urée est de 20:1 à 1:10.

3. Composition antiperspirante selon la revendication 1 ou la revendication 2, où la solution est une solution alcoolique aqueuse comprenant un alcool aliphatique en $C_1$ à $C_3$ en une quantité allant jusqu'à 75% en poids.

4. Composition antiperspirante selon l'une quelconque des revendications précédentes, comprenant de 1 à 25% en poids du composé d'aluminium basique.

5. Composition antiperspirante solide produite en séchant la composition aqueuse selon la revendication 1 ou la revendication 2 et ayant la formule brute:

$$Al_2(OH)_{6-a}X_a \cdot nH_2O \cdot (\text{urée})m$$

où X représente Cl, Br, I ou $NO_3$, a vaut de 0,31 à 1,5, n vaut de 0,3 à 8, et m indique la quantité d'urée.